# EUROPEAN PATENT APPLICATION

(11) **EP 4 674 377 A1**
(43) Date of publication of application: **07.01.2026**
(21) Application number: 25185885.8
(22) Date of filing: 27.06.2025
(51) Int. Cl.: A61C 9/00, A61B 5/00, H04N 23/21, A61B 1/04, A61B 5/0507, A61C 19/04

(54) **ADVANCED ELECTROMAGNETIC SPECTRUM INTRAORAL DIGITAL ECOSYSTEM**

(30) Priority: 03.07.2024 GB 202409591; 20.02.2025 GB 202502465
(71) Applicant: Nulty, Adam Brian, Ixworth, Bury St. Edmunds IP31 2HT (GB)
(72) Inventor: Nulty, Adam Brian, Ixworth, Bury St. Edmunds IP31 2HT (GB)
(74) Representative: Doherty, William

(57) **Abstract**

The Advanced Electromagnetic Spectrum Intraoral Digital Ecosystem (AESIDE) revolutionizes dental scanning technology with its shape-memory polymer tray 10, embedded multi-spectral micro-cameras 18, and real-time AI processing. NIR, MIR, and THz imaging provide comprehensive intraoral views, while secure wireless connectivity and cloud integration enable collaborative treatment planning among dental professionals. Augmented reality enhances user interaction, and environmental features include biodegradable tray sleeves and self-sterilizing technology, ensuring hygiene and sustainability.

## Description

**Field of Invention and Background:** The invention relates to intraoral scanning systems, specifically the AESIDE, which utilizes multi-spectral imaging and advanced AI processing for comprehensive oral cavity visualization. Current intraoral scanners often lack the capability to penetrate tissues effectively or provide real-time processing for immediate diagnostics and treatment planning.

**Summary of the Invention:** AESIDE introduces a shape-memory polymer tray with modular extensions and embedded multi-spectral micro-cameras, enabling detailed imaging using near-infrared (NIR), mid-infrared (MIR), and terahertz (THz) wavelengths. Real-time AI processing and secure wireless connectivity to cloud platforms facilitate collaboration and data sharing among dental professionals. Augmented reality feedback enhances user interaction, and environmental considerations include biodegradable sleeves and self-sterilizing technology.

According to a first aspect of the invention, there is provided an intraoral scanning system, comprising: an impression tray which is formed from a shape-memory polymer adaptable to various mouth shapes and sizes; a plurality of multi-spectral micro-cameras embedded within the impression tray, wherein the plurality of multi-spectral micro cameras has imaging capabilities using at least NIR, MIR, and/or THz wavelengths; and an onboard processing unit capable of implementing AI processing for real-time stitching of images into 3D stereolithography models.

Optionally, the impression tray may comprise a plurality of modular extension segments for adjusting a size thereof.

Optionally, the modular extension segments may securely snap onto the tray, allowing customization for different patient mouth sizes.

Optionally, the modular extension segments of the impression tray may be magnetically attachable for quick adjustment and setup.

Optionally, electrical connections between the plurality of multi-spectral micro-cameras and the onboard processing unit may be embedded into the impression tray.

Optionally, the electrical connectors may be provided on a flexible printed circuit board embedded into the impression tray.

The intraoral scanning system may further comprise a contact interface positioned at a posterior of the impression tray for data transmission and/or powering.

Optionally, the plurality of multi-spectral micro-cameras may be CMOS micro-cameras.

Optionally, the NIR imaging component may penetrate soft tissues for sub-gingival structure visualization without interference, and/or wherein the MIR imaging component distinguishes between enamel and dentin, providing detailed analysis for prosthetic planning, and/or wherein the THz imaging component provides detailed views of both soft and hard tissues, aiding in the detection of dental anomalies.

Optionally, the impression tray may include biocompatible materials ensuring patient comfort and safety.

The intraoral scanning system may further comprise adaptive software algorithms adjusting imaging parameters based on tissue type detected by multi-spectral imaging.

Optionally, the multi-spectral micro-cameras may synchronize to capture images simultaneously for comprehensive intraoral scanning.

The intraoral scanning system may further comprise a secure wireless transmission module transmitting scanned data to a cloud platform suitable for collaborative editing and sharing of 3D models among dental professionals.

Optionally, the cloud platform may employ AI algorithms for real-time analysis of scanned data, providing predictive diagnostics for dental conditions.

Optionally, the AI processing unit may perform error detection and correction ensuring the accuracy of generated 3D models.

The intraoral scanning system may further comprise an augmented reality (AR) headset overlaying real-time scanned data for immediate treatment planning and patient interaction.

The intraoral scanning system may further comprise biodegradable sleeves for the impression tray ensuring hygiene and environmental sustainability.

The intraoral scanning system may further comprise self-sterilizing UV-C LEDs integrated within the tray structure ensuring device sterilization between uses.

Optionally, the impression tray may include a temperature-regulating feature enhancing patient comfort during scanning procedures.

Optionally, the AI processing unit may continuously update and improve its algorithms based on collected data for enhanced scanning accuracy.

Optionally, the shape-memory polymer tray may include a sensor array for monitoring patient vital signs during scanning procedures.

Optionally, the AI processing unit may analyze dental conditions and suggest treatment plans based on historical data stored in the cloud platform.

The intraoral scanning system may further comprise a haptic feedback system integrated into the impression tray providing tactile confirmation of accurate scanning.

Optionally, the shape-memory polymer tray may be adaptable to dental impression materials for capturing traditional impressions alongside digital scans.

The intraoral scanning system may further comprise an automated calibration system ensuring accurate alignment of multi-spectral micro-cameras prior to each scanning session.

### Drawings and Figures:

- Figure 1: Illustration of the shape-memory polymer tray with modular extensions and embedded multi-spectral micro-cameras.
- Figure 2: Diagram showing the synchronization of multi-spectral micro-cameras capturing images simultaneously.
- Figure 3: Schematic of the onboard AI processing unit stitching images into a 3D STL model in real-time.
- Figure 4: Layout of the augmented reality (AR) headset overlaying scanned data onto the patient's mouth.

### Detailed Description of the Drawings:

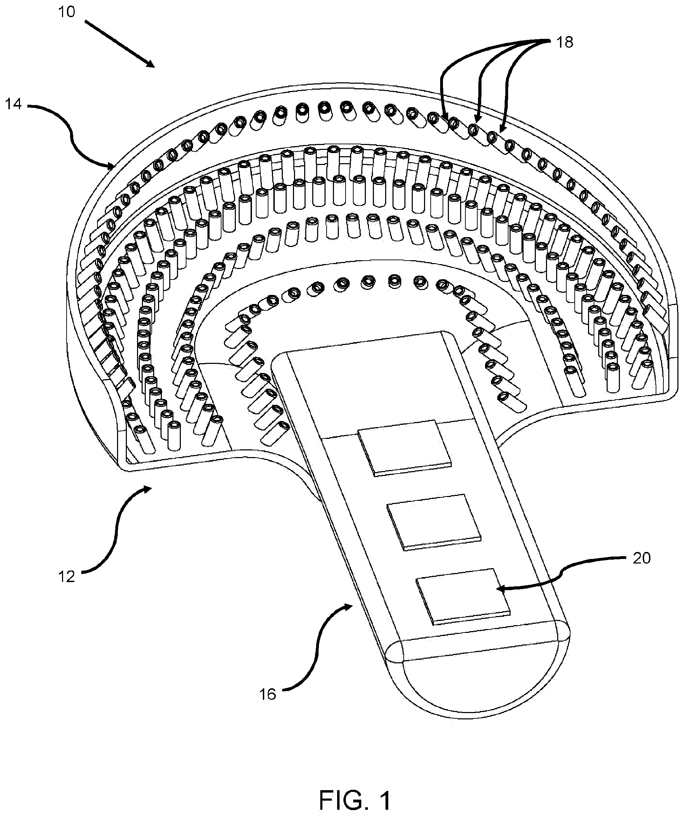

- Figure 1 illustrates the adaptive design of the shape-memory polymer tray with modular extensions, showcasing its ability to conform to different mouth shapes and sizes while housing embedded multi-spectral micro-cameras.

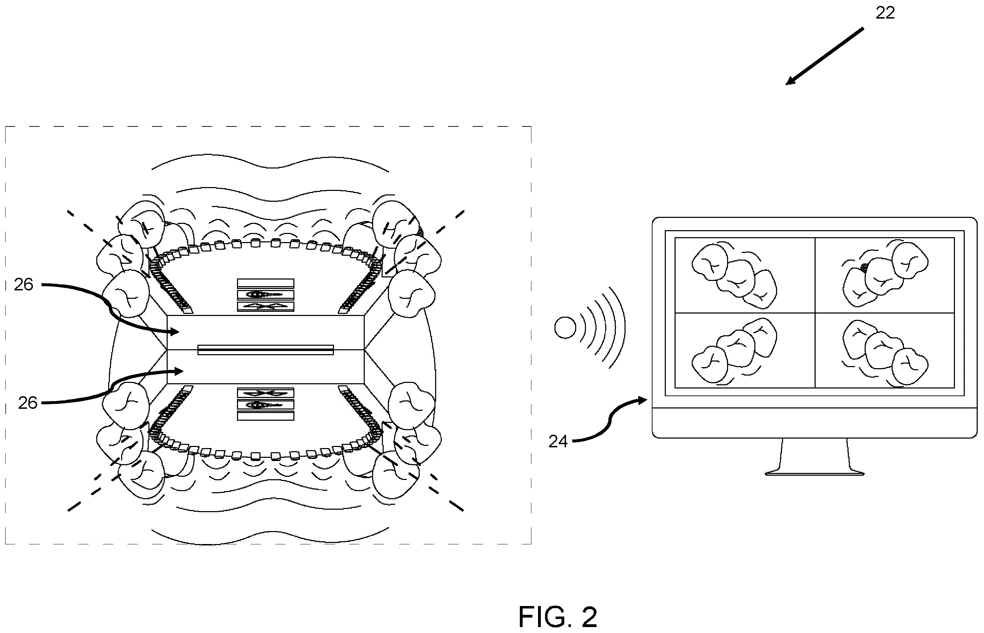

- Figure 2 details the synchronized operation of the multi-spectral micro-cameras within the tray structure, ensuring comprehensive intraoral scanning from various angles simultaneously.

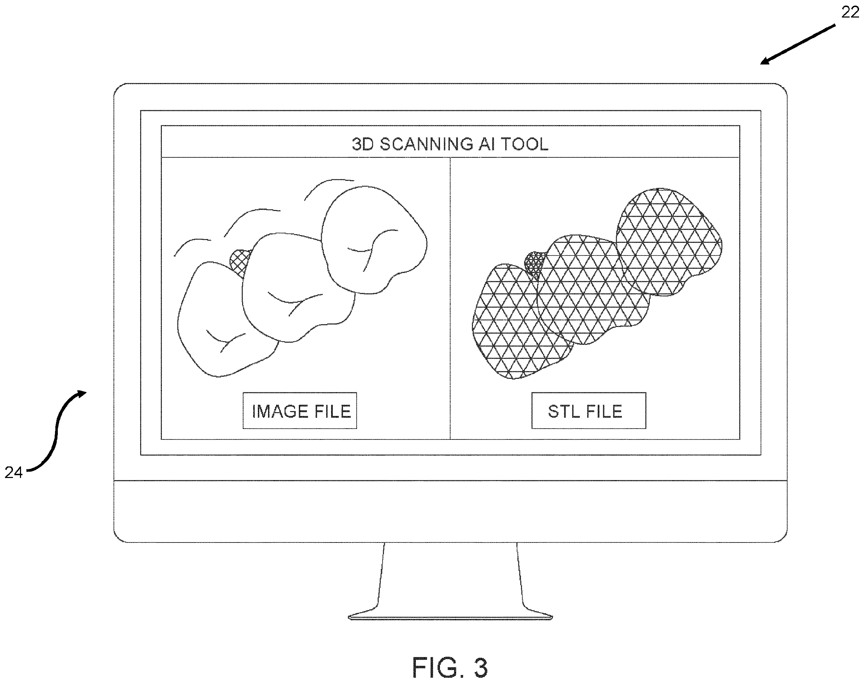

- Figure 3 depicts the onboard AI processing unit coordinating the real-time stitching of images captured by the multi-spectral micro-cameras, generating accurate 3D STL models for immediate analysis and treatment planning.

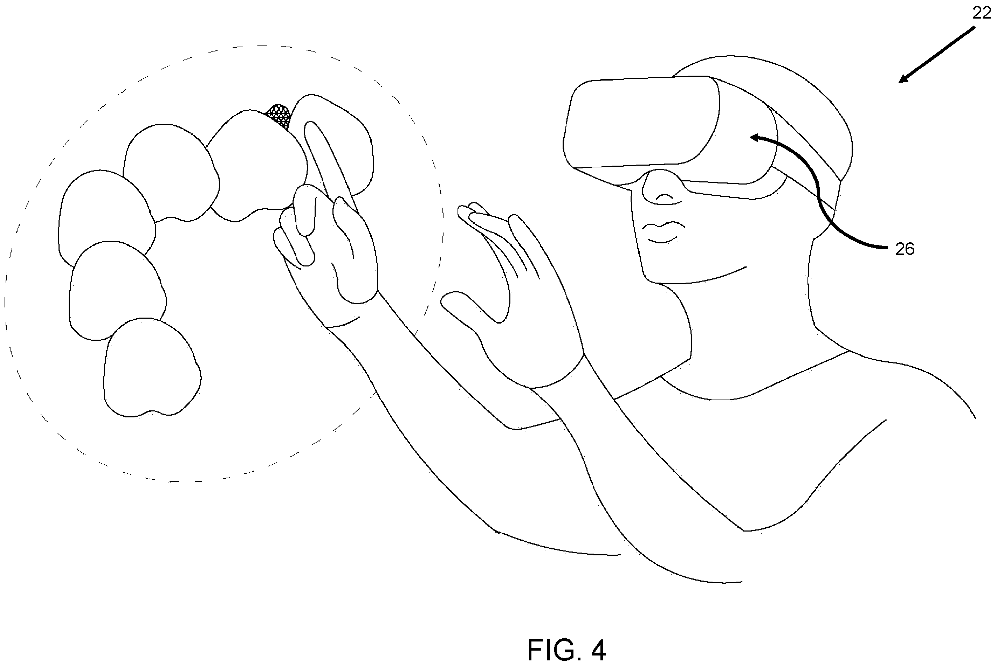

- Figure 4 outlines the augmented reality (AR) headset overlaying real-time scanned data onto the patient's mouth, facilitating immediate treatment decisions and patient interaction during dental procedures.

**Detailed Description of the Invention:** AESIDE comprises a shape-memory polymer tray designed to adapt to various mouth shapes and sizes, enhanced by modular extension segments for customization. Embedded within the tray are hundreds of multi-spectral micro-cameras strategically placed to capture comprehensive intraoral images.

The imaging system includes:
- NIR imaging for sub-gingival visualization and blood vessel detection.
- MIR imaging for distinguishing enamel, dentin, and other tissues.
- THz imaging for detailed views of both soft and hard tissues, aiding in the detection of dental anomalies.

An onboard AI processing unit synchronizes and stitches images from micro-cameras in real-time, generating accurate 3D STL models. Error detection and correction algorithms ensure high-quality scans without manual intervention. Secure wireless transmission sends data to a cloud platform supporting collaborative editing and sharing of 3D models among dental professionals.

Augmented reality integration provides dentists with real-time overlays of scanned data, facilitating immediate treatment decisions and patient engagement. Environmental considerations include biodegradable tray sleeves and self-sterilizing UV-C LEDs, ensuring hygiene and reducing environmental impact.

Figure 1 shows the impression tray, referenced at 10. It is preferably formed having a body 12 from a shape memory polymer adaptable to various mouth sizes and shapes. The body 12 has a bite region 14 and an additional housing component 16.

There is a plurality of multi-spectral micro-cameras 18 embedded within the impression tray 10. The plurality of multi-spectral micro cameras 18 has imaging capabilities using at least NIR, MIR, and/or THz wavelengths. The plurality of multi-spectral micro-cameras 18 are preferably embedded in the bite region 14.

There is also an onboard processing unit capable of implementing AI processing for real-time stitching of images into 3D stereolithography (STL) models. In the depicted embodiment, the processing unit would be embedded in the additional housing component 16. Electrical connections between the plurality of multi-spectral micro-cameras 18 and the onboard processing unit are embedded into the impression tray 10, for example, in the form of a flexible printed circuit board embedded into the impression tray 10. A contact interface 20 may be provided positioned at a posterior of the impression tray 10 for data transmission and/or powering. An automated calibration system may be provided ensuring accurate alignment of multi-spectral micro-cameras 18 prior to each scanning session.

Whilst a unitary impression tray 10 is shown, it may be possible to provide an impression tray having a plurality of modular extension segments for adjusting a size thereof. Such modular extension segments may securely snap onto the tray, allowing customization for different patient mouth sizes.

One or more biodegradable sleeves may be provided for the impression tray 10 ensuring hygiene and environmental sustainability.

Self-sterilizing UV-C LEDs could also be integrated within the body 12ensuring device sterilization between uses.

A haptic feedback system may be integrated into the impression tray 10 providing tactile confirmation of accurate scanning. This could be provided in the form of an onboard vibration generator.

The impression tray 10 may also include a temperature-regulating feature enhancing patient comfort during scanning procedures. This could be provided via a thermally active member embedded in the body 12.

The impression tray 10 may include a sensor array for monitoring patient vital signs during scanning procedures.

The impression tray 10 may be adaptable to dental impression materials to enable it to capture traditional physical dental impressions in addition to the digital scans created.

To use the impression tray 10 as part of an intraoral scanning system 22, such as that shown in Figure 2, one or more impression trays 10 is inserted into the patient's mouth, and multispectral imaging of the mouth can be taken and transmitted to an external device 24. Adaptive software algorithms may be provided for adjusting imaging parameters based on tissue type detected by multi-spectral imaging. The multi-spectral micro-cameras may synchronize to capture images simultaneously for comprehensive intraoral scanning.

In order to communicate with an external device 24, a secure wireless transmission module may be provided for transmitting scanned data to a cloud platform suitable for collaborative editing and sharing of 3D models among dental professionals. The cloud platform may employ AI algorithms for real-time analysis of scanned data, providing predictive diagnostics for dental conditions.

The AI processing unit continuously updates and improves its algorithms based on collected data for enhanced scanning accuracy. The AI processing unit may also analyze dental conditions and suggests treatment plans based on historical data stored in the cloud platform.

The AI processing unit performs error detection and correction ensuring the accuracy of generated 3D model, as shown in Figure 3, where the STL model is generated based on the data received.

As shown in Figure 4, the intraoral scanning system 22 may also comprise an augmented reality (AR) headset 26 overlaying real-time scanned data for immediate treatment planning and patient interaction.

### Key Features:

### 1. Shape-Memory Polymer Tray:

° **Adaptive Tray Design:** The impression tray is made from a shape-memory polymer that conforms perfectly to any mouth shape and size. This adaptive design provides a comfortable fit for all patients and ensures accurate scanning.
° **Modular Extension Segments:** The tray can be elongated with snap-on modules made of the same shape-memory material, providing a seamless and secure fit for larger mouths.
° Any commercially available shape-memory material suitable for intraoral applications can be considered, including, but not limited to, polyurethane-based shape-memory polymers, or polycaprolactone (PCL)-based polymers.
° The shape-memory polymer should have a transition temperature in the range of 30-50°C, which aligns with intraoral conditions and allows for easy adaptation when please in the mouth.
° Thermomechanical training can be used in the manufacture of the shape-memory polymer, allowing the impression tray to return to a predefined shape after deformation.

### 2. Hundreds of Multi-Spectral Micro-Cameras:

**° Embedded Camera Array:** Integrate hundreds of multi-spectral micro-cameras within the tray structure, positioned to cover all angles of the oral cavity. Each camera captures high-resolution images simultaneously.
° **Multi-Spectral Imaging:** Utilize a range of wavelengths from the electromagnetic spectrum, including near-infrared (NIR), mid-infrared (MIR), and terahertz (THz) radiation, to see through blood and soft tissue and capture detailed information about underlying structures.
° Examples of such micro-camera includes complementary metal-oxide-semiconductor (CMOS)-based multispectral sensors in the sub-1mm size range, which are commercially available and used in minimally invasive medical imaging.
∘ Electrical connections can be achieved using flexible printed circuit boards integrated within the polymer matrix, ensuring connectivity whilst maintaining the flexibility of impression tray.
∘ Data transmission and power supply can be managed through contacts, such as gold-plated contact pads, positioned at the posterior of the impression tray for external interfacing.

### 3. Holistic Multi-Spectral Imaging:

**∘ Near-Infrared (NIR) Imaging:** NIR wavelengths (700-1400 nm) are used to penetrate soft tissues and visualize sub-gingival structures and blood vessels. NIR is particularly effective in providing detailed images of soft tissue without interference from blood.
∘ **Mid-Infrared (MIR) Imaging:** MIR wavelengths (1400-3000 nm) offer deeper tissue penetration and are capable of distinguishing between different types of tissues, such as enamel and dentin.
∘ **Terahertz (THz) Imaging:** THz radiation (0.1-10 THz) can penetrate both soft and hard tissues, providing a detailed view of the entire oral cavity, including bone structures. This wavelength is particularly useful for identifying dental caries and other anomalies beneath the surface.

### 4. Real-Time AI-Enhanced Processing:

**∘ Onboard AI Processing Unit:** Equipped with an advanced AI processing unit that stitches together the images from all cameras in real-time, creating a comprehensive 3D STL model within seconds.
∘ **Error Detection and Correction:** The AI algorithms automatically detect and correct errors, ensuring a flawless 3D model. The system also identifies any areas that require rescanning.
∘ Examples of AI processing units known in the art include the NVIDIA Jetson Nano, Google Edge TPU, or Qualcomm Snapdragon XR2. These are commercially available processors capable of real-time, low-power AI inference.
∘ Such processors are capable of real-time stitching of multi-spectral images from the cameras, enabling 3D reconstruction without requiring cloud connectivity.
∘ Pre-trained models can be loaded onto the processing unit, removing the need to generate training data in situ.

### 5. Wireless Connectivity and Cloud Integration:

∘ **Secure Wireless Transmission:** Use advanced wireless technology to transmit the STL files securely to a cloud platform, where they can be accessed and analyzed by dental professionals.
∘ **Cloud-Based Collaboration:** Enable real-time collaboration between dentists, orthodontists, and dental labs through a cloud-based platform that supports sharing and editing of the 3D models.

### 6. Immersive Augmented Reality (AR) Feedback:

∘ **AR Integration for Dentists:** Incorporate an AR headset for dentists that overlays the scanned data onto the patient's mouth in real-time. This allows for immediate assessment and adjustments during the scanning process.
∘ **Patient Engagement:** Use AR to show patients their 3D models and explain treatment plans interactively, improving patient understanding and satisfaction.

### 7. Environmental and Hygienic Considerations:

∘ **Biodegradable Sleeves:** Use single-use, biodegradable sleeves for the tray to ensure hygiene and reduce environmental impact.
∘ **Self-Sterilizing Technology:** Integrate self-sterilizing UV-C LEDs within the tray to automatically sterilize the device between uses.

### Differentiation and Impact:

### 1. Revolutionary Imaging and Processing:

∘ **Multi-Spectral Scanning:** The combination of NIR, MIR, and THz imaging provides comprehensive visualization of the oral cavity, capturing detailed information about both soft and hard tissues.
∘ **Real-Time AI Processing:** Real-time error detection and correction by onboard AI ensures high-quality 3D models without the need for manual intervention.

### 2. Adaptive and Comfortable Design:

∘ **Shape-Memory Polymer:** The adaptive tray design ensures comfort and accuracy for all patients, providing a better experience compared to rigid trays.
∘ **Modular Extensions:** The ability to adjust the size of the tray with modular extensions makes it versatile and suitable for a wide range of patients.

### 3. Enhanced User Interaction:

∘ **AR Integration:** Augmented reality feedback for dentists and patients creates an immersive and interactive experience, enhancing the diagnostic and consultation process.
∘ **Cloud Collaboration:** Secure wireless connectivity and cloud integration facilitate real-time collaboration and streamline the workflow from scan to treatment.

### 4. Sustainable and Hygienic:

∘ **Biodegradable and Self-Sterilizing Features:** The use of biodegradable sleeves and self-sterilizing technology addresses hygiene concerns and reduces environmental impact, making the device both patient-friendly and eco-friendly.

### Potential Applications:

- **Orthodontics:** Enhanced accuracy in creating detailed 3D models for planning and adjusting braces and aligners.
- **Prosthodontics:** Precision in designing dental prosthetics such as crowns, bridges, and dentures.
- **Implantology:** Accurate planning and placement of dental implants with comprehensive tissue and bone data.
- **Diagnostics:** Improved diagnostic capabilities with detailed multi-spectral imaging of the oral cavity.

### Technical Feasibility and Considerations:

### 1. Micro-Camera Integration:

∘ **Miniaturization and Placement:** Ensure the multi-spectral micro-cameras are sufficiently miniaturized and strategically placed to capture comprehensive data without causing discomfort.
∘ **Synchronized Operation:** Develop robust synchronization technology to ensure all cameras capture images simultaneously.

### 2. Advanced AI Processing:

∘ **High-Performance AI Chips:** Use high-performance AI chips capable of real-time image processing and error correction.
∘ **Training and Optimization:** Continuously train and optimize AI algorithms for various dental conditions and scenarios.

### 3. AR and Cloud Integration:

∘ **Seamless Connectivity:** Implement secure and fast wireless connectivity for real-time data transmission to the cloud.
∘ **User-Friendly AR Interface:** Develop an intuitive AR interface for dentists to enhance the user experience and improve diagnostic accuracy.

By integrating these innovative features and focusing on advanced multi-spectral imaging, real-time processing, and user interaction, the Advanced Electromagnetic Spectrum Intraoral Digital Ecosystem (AESIDE) would set a new standard in dental scanning technology, offering unmatched speed, accuracy, and patient comfort.

**Conclusion, Ramifications, and Scope:** AESIDE represents a paradigm shift in dental scanning technology, offering unparalleled accuracy, real-time diagnostics, and enhanced patient interaction through multi-spectral imaging, AI processing, and augmented reality integration. The invention's adaptive design, secure cloud connectivity, and environmental considerations set new standards in dental care, promising improved treatment outcomes, workflow efficiency, and patient satisfaction.

This structured patent application effectively communicates the innovative features and potential impact of AESIDE in dental practice, ensuring comprehensive protection and differentiation in the marketplace.

## Claims

1. An intraoral scanning system (22), comprising:
∘ an impression tray (10) which is formed from a shape-memory polymer adaptable to various mouth shapes and sizes;
∘ a plurality of multi-spectral micro-cameras (18) embedded within the impression tray, wherein the plurality of multi-spectral micro cameras (18) has imaging capabilities using at least NIR, MIR, and/or THz wavelengths; and
∘ an onboard processing unit capable of implementing AI processing for real-time stitching of images into 3D stereolithography models.

2. The intraoral scanning system (22) of claim 1, wherein the impression tray (10) comprises a plurality of modular extension segments for adjusting a size thereof.

3. The intraoral scanning system (22) of any one of the preceding claims, wherein electrical connections between the plurality of multi-spectral micro-cameras (18) and the onboard processing unit are embedded into the impression tray (10).

4. The intraoral scanning system (22) of claim 3, wherein the electrical connectors are provided on a flexible printed circuit board embedded into the impression tray (10).

5. The intraoral scanning system (22) of any one of the preceding claims, further comprising a contact interface positioned at a posterior of the impression tray (10) for data transmission and/or powering.

6. The intraoral scanning system (22) of any one of the preceding claims, wherein the NIR imaging component penetrates soft tissues for sub-gingival structure visualization without interference, and/or wherein the MIR imaging component distinguishes between enamel and dentin, providing detailed analysis for prosthetic planning, and/or wherein the THz imaging component provides detailed views of both soft and hard tissues, aiding in the detection of dental anomalies.

7. The intraoral scanning system (22) of any one of the preceding claims, further comprising adaptive software algorithms adjusting imaging parameters based on tissue type detected by multi-spectral imaging.

8. The intraoral scanning system (22) of any one of the preceding claims, further comprising a secure wireless transmission module transmitting scanned data to a cloud platform suitable for collaborative editing and sharing of 3D models among dental professionals.

9. The intraoral scanning system (22) of claim 8, wherein the cloud platform employs AI algorithms for real-time analysis of scanned data, providing predictive diagnostics for dental conditions.

10. The intraoral scanning system (22) of any one of the preceding claims, further comprising an augmented reality (AR) headset overlaying real-time scanned data for immediate treatment planning and patient interaction.

11. The intraoral scanning system (22) of any one of the preceding claims, further comprising biodegradable sleeves for the impression tray (10) ensuring hygiene and environmental sustainability and/or self-sterilizing UV-C LEDs integrated within the tray structure ensuring device sterilization between uses.

12. The intraoral scanning system (22) of any one of the preceding claims, wherein the impression tray (10) includes a temperature-regulating feature enhancing patient comfort during scanning procedures and/or includes a sensor array for monitoring patient vital signs during scanning procedures.

13. The intraoral scanning system (22) of any one of the preceding claims, wherein the AI processing unit analyzes dental conditions and suggests treatment plans based on historical data stored in the cloud platform.

14. The intraoral scanning system (22) of any one of the preceding claims, further comprising a haptic feedback system integrated into the impression tray (10) providing tactile confirmation of accurate scanning.

15. The intraoral scanning system (22) of any one of the preceding claims, wherein the shape-memory polymer tray (10) is adaptable to dental impression materials for capturing traditional impressions alongside digital scans.
